# FASCICULE DE BREVET EUROPEEN

(11) **EP 4 395 642 B1**
(45) Date de publication et mention de la délivrance du brevet: **02.07.2025**
(21) Numéro de dépôt: 22762129.9
(22) Date de dépôt: 29.07.2022
(51) Int. Cl.: A61B 5/145, A61B 5/00

(54) **DISPOSITIF DE SURVEILLANCE CORPORELLE À CONTACTS AMÉLIORÉS**
KÖRPERÜBERWACHUNGSVORRICHTUNG MIT VERBESSERTEN KONTAKTEN
BODY MONITORING DEVICE COMPRISING IMPROVED CONTACTS

(30) Priorité: 30.07.2021 FR 2108338
(43) Date de publication de la demande: 10.07.2024
(73) Titulaire: WIZP AS, 3183 Horten (NO)
(72) Inventeur: PIERART, Luc, 94800 Villejuif (FR); BADIOLA, Florian, 75006 Paris (FR)
(74) Mandataire: Regimbeau
(86) Numéro de dépôt international: PCT/FR2022/051526
(87) Numéro de publication internationale: WO 2023/007102

(56) Documents cités:
- EP-A1- 3 430 993
- EP-A1- 3 603 508
- EP-A2- 3 136 954
- US-A1- 2021 128 042

## Description

### DOMAINE TECHNIQUE

L'invention concerne un dispositif de surveillance corporelle et en particulier un dispositif de surveillance corporelle par une analyse de liquide corporel, typiquement interstitiel au moyen de microaiguille(s).

### ETAT DE LA TECHNIQUE

La surveillance de nombreuses maladies chroniques connues chez l'humain nécessite un relevé quotidien de paramètres biochimiques. Un niveau de concentration d'un analyte corporel dans un fluide corporel de l'organisme, par exemple dans le plasma sanguin ou dans le liquide interstitiel des cellules de l'organisme, peut être relevé.

A titre d'exemple répandu, le suivi du diabète chez un patient nécessite un relevé précis quotidien de la glycémie du patient.

Une solution répandue pour réaliser le suivi du diabète consiste à réaliser une ponction, par exemple au bout du doigt, pour faire perler une goutte de sang, puis à réaliser une mesure quotidienne de glycémie dans la goutte de sang ainsi obtenue.

Des systèmes de suivi ont été proposés pour se passer de la nécessité d'une ponction manuelle, de sorte à rendre la mesure de glycémie moins laborieuse et moins invasive. On parle de systèmes CGM, pour « Continuous Glucose Monitoring ». Certains de ces systèmes CGM réalisent, à intervalles réguliers, une mesure de glycémie au niveau du liquide interstitiel entre les cellules de la peau. La glycémie du liquide interstitiel est très proche de la glycémie du plasma sanguin. Les mesures au niveau du liquide interstitiel permettent un suivi simple et peu invasif de la glycémie des patients ; ces mesures peuvent être réalisées à l'aide de capteurs à aiguille, en transcutané, ou de manière non invasive, comme par exemple en iontophorèse ou en implantable avec une mesure par chimio-fluorescence.

La demande internationale publiée sous le numéro WO 2018/104647 décrit un système de surveillance corporelle, utilisable notamment pour le suivi de la glycémie. Ce système de surveillance inclut une montre électronique attachable au poignet à l'aide d'un bracelet. La montre comporte un boîtier, dans lequel s'insère une capsule amovible interchangeable comprenant un capteur à micro-aiguilles. Le capteur est commandé de manière automatique par l'électronique du boîtier, pour réaliser une mesure transcutanée. La mesure de glycémie par le capteur est une mesure électrochimique.

Le dispositif comprend des connecteurs qui permettent de connecter électriquement et physiquement le capteur au boitier, en particulier chaque connecteur comprend un partie male disposée sur le boitier et une partie femelle disposée sur le capteur. En particulier, les connecteurs sont à ressorts en ce que la partie male est élastique lorsqu'elle s'enfonce dans la partie femelle. De tels connecteurs ressorts permettent d'améliorer la connexion notamment lorsque la connexion entre le capteur et le boitier est fluctuante compte tenu des différences d'enfoncements des aiguilles dans la peau par exemple ou compte tenu des mouvements du poignet de l'utilisateur.

Ainsi, la connexion entre le capteur et le boitier peut être imparfaite ce qui peut nuire aux mesures et donc à la surveillance, cette connexion imparfaite générant du bruit de mesure. Un dispositif alternatif est divulgué dans le document EP 3136954 A2.

En outre, on connait des connecteurs de contact physique, en opposition au contact radio, qui comprennent deux fonctions associées : une fonction de maintien mécanique forte et stable (clippage, serrage en force) et une fonction de connexion électrique (contact métal). Le fait de ne pas avoir cette fonction de maintien mécanique forte est problématique.

En effet dans ce type de connecteur, la difficulté réside dans le fait que la fonction de tenue mécanique n'est pas fixe et forte, mais lâche et fluctuante puisqu'elle dépend de paramètres extérieurs (évolution du diamètre du poignet dans la journée, souplesse du bracelet, chocs, etc.).

### EXPOSE DE L'INVENTION

L'invention améliore la connexion entre un capteur et un boitier lorsque ces derniers sont connectés ensemble de manière lâche au moyen de connecteurs ressorts c'est-à-dire lorsqu'il n'y pas de de maintien mécanique au niveau des connecteurs.

A cet effet, l'invention propose un dispositif de surveillance corporelle suivant la revendication 1, le dispositif comprenant :
- un boitier comprenant une face inférieure ;
- un capteur comprenant une face inférieure destinée à être disposé sur un membre d'un utilisateur et une face supérieure disposée en face de la face inférieure du boitier, le capteur et le boitier étant séparable ou détachable l'un de l'autre à la demande, aucune liaison mécanique ne retenant le boitier au capteur fermement ensemble, le boitier étant en contact électrique et physique de manière lâche avec le capteur par l'intermédiaire d'une pluralité de connecteurs, chaque connecteur comprenant une partie femelle et une partie male ressort de manière à ce que la partie male exerce une force sur la partie femelle tendant à écarter le boitier du capteur ou inversement ; le dispositif comprenant en outre
- des moyens d'attache du boitier à un membre de l'utilisateur, lesdits moyens d'attache étant configurés pour appliquer une force de serrage afin de compenser la force écartant le boitier du capteur lorsque celui-ci est attaché au membre de l'utilisateur ;
le dispositif étant tel que les connecteurs sont répartis par rapport à une zone de répartition définie sur la face supérieure du capteur ou sur la face inférieure du boitier, plus de 70% des connecteurs étant répartis de manière symétrique dans chaque moitié de la zone de répartition par rapport au centre du boitier qui correspond à l'épicentre de la force exercée par les moyens d'attache.

L'invention est avantageusement complétée par les caractéristiques suivantes, prises seules ou en une quelconque de leur combinaison techniquement possible :
- les parties males et les parties femelles de chaque connecteur sont réparties de manière identique et complémentaire sur leur face respective ;
- les parties males et les parties femelles de chaque connecteur ne sont pas réparties de manière identique et complémentaire sur leur face respective ;
- le dispositif comprend six à vingt connecteurs, de préférence dix ou douze connecteurs répartis dans la zone de répartition, la zone de répartition étant polygonale ou de forme ovoïde par exemple délimitée par un cercle, une ellipse ou un ovale, ou est une portion d'un polygone ;
- le dispositif comprend en outre des zones comprenant une partie male et une partie femelle, la partie femelle étant telle que lorsque la partie male est introduite dans la partie femelle, la partie male n'exerce pas de force contre la partie femelle ;
- les connecteurs sont reliés entre eux par groupe de deux à sept connecteurs, de préférence trois à quatre connecteurs ;
- chaque connecteur comprend une partie femelle formée dans l'épaisseur du capteur et une partie male en saillie de la face inférieure du boitier ;
- les parties males sont des broches pogos configurées pour autoriser une amplitude comprise entre 50 µm et 2mm inclus lorsque le capteur est en contact avec le boitier ;
- le dispositif comprend une liaison amovible entre le capteur et le boitier, la liaison étant de préférence constituée par des parties aimantées complémentaires agencées sur le capteur et/ou le boitier ;
- le capteur est configuré pour mesurer une grandeur physiologique ou biochimique de l'utilisateur, et comprend de préférence au moins une microaiguille adaptée pour être insérée fans la peau de l'utilisateur.

La répartition des connecteurs améliore la connexion dans le cadre d'absence de maintien mécanique procuré par les connecteurs.

En effet, compte tenu que ce sont les moyens d'attaches qui permettent de lier le capteur et le boitier en les rapprochant et donc en venant contre la force des parties males, une bonne répartition des efforts compense un serrage des moyens d'attaches imparfait et/ou des mouvements du poignet de l'utilisateur.

### PRESENTATION DES FIGURES

D'autres caractéristiques, buts et avantages de l'invention ressortiront de la description qui suit, qui est purement illustrative et non limitative, et qui doit être lue en regard des dessins annexés sur lesquels :
- la figure 1 illustre un dispositif en coupe selon un mode de réalisation de l'invention ;
- la figure 2 illustre un dispositif en perspective selon un mode de réalisation de l'invention ;
- la figure 3 illustre un dispositif dans une autre perspective selon un mode de réalisation de l'invention ;
- la figure 4 illustre schématiquement un connecteur selon un mode de réalisation utilisé par le dispositif de l'invention ;
- la figure 5 illustre schématiquement un connecteur selon un mode de réalisation utilisé par le dispositif de l'invention ;
- les figures 6a et 6b illustrent une répartition des connecteurs d'un dispositif selon l'invention selon un mode de réalisation ;
- les figures 7a et 7b illustrent une répartition des connecteurs d'un dispositif selon l'invention selon un mode de réalisation ;
- les figures 8a et 8b illustrent une répartition des connecteurs d'un dispositif selon l'invention selon un mode de réalisation.

Sur l'ensemble des figures les éléments similaires portent des références identiques.

### DESCRIPTION DETAILLEE

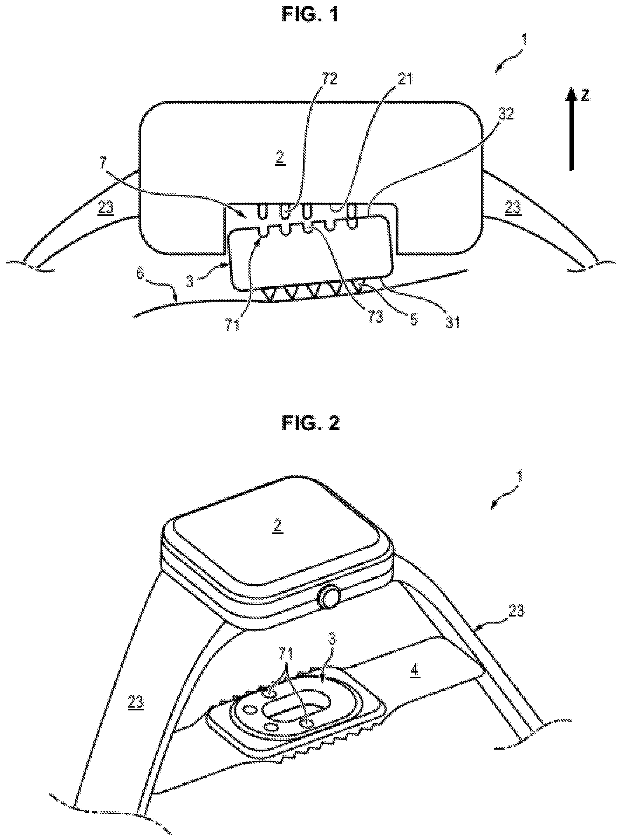

En relation avec les **figures 1, 2** et **3****,** un dispositif 1 de surveillance corporelle comprend un boitier 2, un capteur 3, un patch adhésif 4.

Par surveillance corporelle, on entend la vérification de constantes biochimiques d'une personne porteuse du dispositif, typiquement la concentration en une protéine, une hormone, un marqueur, en oxygène, en nutriments, etc., par exemple dans un liquide corporel de la personne. A titre d'exemple on peut citer la glycémie. L'homme du métier pourra aussi surveiller si besoin d'autres grandeurs physiques corporelles telles que la température, l'hydratation, etc.

On considère de manière préférée mais non limitative que la constante biochimique à surveiller est la concentration en glucose (ou glycémie) au sein du liquide interstitiel de la peau. La glycémie du liquide interstitiel est considérée comme représentative de la glycémie du plasma sanguin.

On considère ici que le liquide corporel est du liquide interstitiel mais on peut aussi considérer d'autres liquides corporels tels que le sang.

Le capteur 3 est dans ce cas un capteur à aiguilles prévu pour fournir une mesure de courant électrique au sein du liquide interstitiel du porteur du dispositif 1. Des aiguilles 5 sont avantageusement disposées sur une face interne 31 du capteur 3. Cette face interne 31 est destinée à être placée sur la peau 6 du porteur. Les aiguilles 5 sont avantageusement des micro-aiguilles. Le capteur 3 comporte de préférence entre quatre et cinquante micro-aiguilles voire quatre cent microaiguilles. Bien entendu, un nombre de différent peut être considéré sans que cela ne limite la description de l'invention ici faite.

On entend par micro-aiguille, une aiguille présentant une hauteur faible de préférence entre 10 µm et 1000 µm, de préférence entre 0,3 mm et 0,8mm. La hauteur des micro-aiguilles est suffisamment faible pour éviter le contact avec un nerf de douleur mécanique du porteur lorsque le dispositif est porté.

Les microaiguilles 5 permettent de mesurer ou prélever le liquide corporel.

Les microaiguilles 5 sont creuses lorsqu'il s'agit de prélever du liquide soit pleine pour analyser directement le liquide. Lorsqu'il s'agit de prélever le liquide, les microaiguilles permettent l'extraction de liquide interstitiel du derme de façon indolore sans perlement de sang et l'envoie vers un capteur logé dans le boitier 2. Lorsqu'il s'agit d'analyser du liquide, les microaiguilles ne prélèvent pas de liquide et intègrent le capteur sur leur surface sous la forme d'un revêtement tel qu'un matériau biochimique apte à réagir avec l'analyse à effectuer sur le liquide.

De manière avantageuse, le capteur 3 comprend plusieurs microaiguilles qui consistent en un réseau de microaiguilles en ce qu'elles sont électriquement connectées entre elles par groupe. Les microaiguilles transpercent la peau pour venir au contact du liquide interstitiel lorsque le capteur est au contact de la peau.

Comme illustré sur la figure 2, le capteur 3 est assemblé au patch adhésif 4 et peuvent ensemble constituer une capsule. Le capteur 3 peut aussi être amovible par rapport au patch 4. Une telle capsule est avantageusement montée amovible avec le boitier 2. En particulier, la capsule et donc le capteur 3 s'engage de manière préférée dans une cavité 21 du boitier 2 située sur sa face destinée à être en contact avec la peau. Le capteur 3 comprend en outre une face externe 32 opposée à la face interne 31.

Le capteur 3 ici illustré est de forme circulaire avec un orifice central 33 mais elle peut prendre d'autres formes : rectangulaire, oblongue, ellipsoïdale avec ou sans orifice central. L'orifice central 33 permet de positionner correctement le capteur 3 dans la cavité 21 du boitier qui comprend un plot central de positionnement (non représenté).

Le capteur 3 comprend donc des éléments qui permettent de prélever le liquide soit d'amener les signaux détectés par chaque microaiguille vers le boitier 2 pour traitement (non décrit ici).

Le capteur 3 peut prendre la forme d'une plaque de plastique, d'un circuit imprimé (rigide ou flexible en silicium), d'une plaque en métal non conducteur comme par exemple de l'aluminium.

Le patch adhésif 4 est adapté pour être collé à la peau et supporte le capteur 3 et permet de détacher le boitier 2 sans enlever le capteur 3 en le gardant collé au corps. Une telle configuration permet d'éviter d'enlever le capteur pour certaines opérations qui n'implique que le boitier : rechargement de la batterie, réparation, remplacement, extraction des données vers un ordinateur.

Le boitier 2 est avantageusement en forme de boitier de montre et comprend des moyens d'attaches 23 du dispositif au poignet d'un utilisateur. Il s'agit notamment d'un bracelet adapté pour entourer le poignet d'un utilisateur. Le boitier 2 loge plusieurs éléments permettant d'analyser ou d'extraire du liquide interstitiel. A ce titre, on pourra se référer au document WO 2019/141743 au nom du déposant qui décrit en détail la mesure et la détection d'une grandeur physique à partir de microaiguilles au contact d'un liquide corporel pouvant être prélevé ou non.

Le contact électrique entre le capteur 3 et le boitier 2 est assuré par l'intermédiaire de plusieurs connecteurs 7 (ou connecteurs ressorts). Ces connecteurs 7 permettent de connecter le capteur 3 au boitier 2. En effet, le boitier 2 doit pouvoir récupérer des informations issues du capteur 3 et doit pouvoir également l'alimenter. Les connecteurs 7 comme déjà mentionné ne permettent pas de lier mécaniquement le capteur 3 au boitier 2.

Chaque connecteur 7 comprend une partie femelle 71 comprenant un orifice 73 formé par exemple dans l'épaisseur du capteur 2 et une partie male 72 en saillie d'une face inférieure 21 du boitier 2. On précise qu'une face inférieure sera plus proche du poignet qu'une face supérieure.

L'orifice 73 est avantageusement concave par rapport à la face supérieure 32 du capteur 3 depuis laquelle il est formé.

Le connecteur 7 est à ressort en ce que la partie male 72 lorsqu'elle s'enfonce dans la partie femelle subit un mouvement. En particulier, la partie male du fait qu'elle comprend une partie élastique (broche ressort ou lame ressort) est configurée pour exercer une force sur le capteur tendant à écarter le boitier 2 du capteur 3 lorsqu'ils sont reliés entre eux.

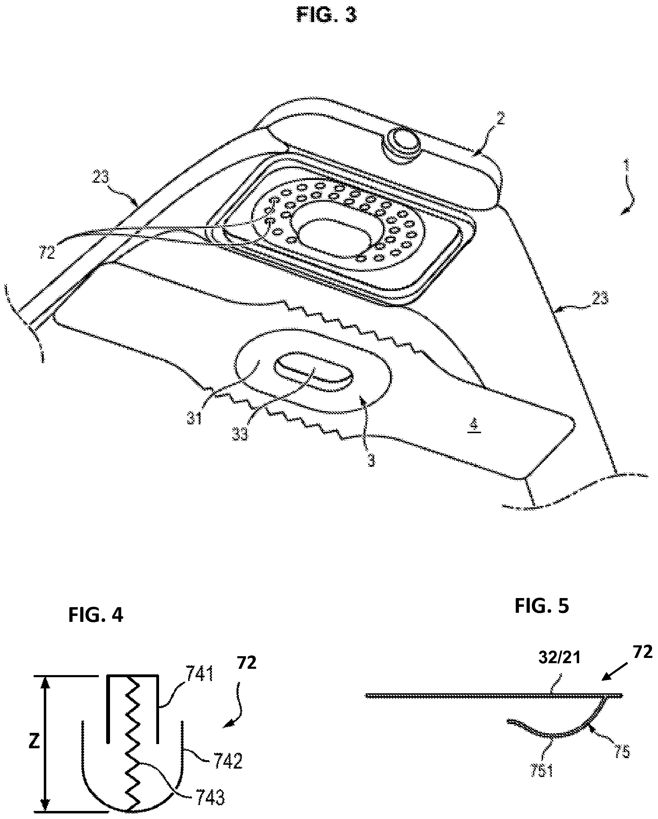

De manière préférée, comme illustré sur la **figure 4****,** la partie male 72 est une broche pogo et comprend une partie fixe 741 et une partie mobile 742 par rapport à la partie fixe 741, la partie male 72 étant configurée pour permettre un déplacement de la partie mobile 742 par rapport à la partie fixe 741 selon une direction Z de translation perpendiculaire à la face inférieure 21 du boitier 2. La partie mobile 742 est coaxiale à la partie fixe 741. En outre, un élément élastique 743 tel qu'un ressort est fixé à l'intérieur entre la partie fixe 741 et la partie mobile. Un tel ressort est un ressort de compression de sorte que sans sollicitation de la broche selon la direction Z la partie mobile est plus éloignée de la partie fixe que sans sollicitation. Dans le cas d'une broche pogo le ressort pour être compressé nécessite une force de 1 newton.

Selon une réalisation préférée, la partie fixe présente une hauteur de 2,5 mm et la partie mobile présente une hauteur de 0,6 mm. Toujours selon cette réalisation préférée, le ressort présente une raideur tell qu'il permet à la partie mobile de s'insérer dans la partie fixe d'environ 0,3 mm. En particulier, chaque broche pogo autorise une translation d'une amplitude comprise entre 50 µm et 2 mm inclus lorsque le capteur est en contact avec le boitier.

De manière alternative, comme illustré sur la **figure 5** la partie male 72 est constituée d'une lame métallique ressort s'étendant depuis la surface inférieure 21 du boitier 2. La lame métallique 75 est avantageusement conformée pour présenter une partie 751 configurée pour être introduite dans ou sur la partie femelle 71 constituée par un orifice 73 (voir la figure 1). Cette partie 751 qui est destinée à être introduite dans l'orifice 73 est avantageusement sphérique. En outre, la lame 75 est en métal conducteur.

Si l'utilisation de tels connecteurs permet de faciliter la connexion électrique entre le boitier et le capteur et de rattraper des mouvements du poignet ou bien un serrage imparfait, il est nécessaire de pouvoir compenser la force qu'ils exercent.

En effet, le boitier 2 et le capteur 3 sont connectés ensembles de manière lâche en ce qu'aucune liaison mécanique ne les retient fermement ensemble.

Pour ce faire, le bracelet 23 permet de compenser cette force mais seul ce n'est pas suffisant. En effet, en fonction du serrage de ce dernier la répartition des efforts qu'il exerce sur le boitier 2 ne sera pas forcément optimale.

Ainsi, on prévoit que les connecteurs soient répartis par rapport à une zone de répartition ZR définie sur la face supérieure du capteur 3 et/ou sur la face inférieure du boitier 2 telles que plus de 70% des connecteurs sont répartis de manière symétrique dans chaque moitié de la zone de répartition ZR par rapport au centre du boitier 2 qui correspond à l'épicentre de la force exercée par le bracelet 23. Ce sont les parties males et femelles des connecteurs qui sont répartis sur la zone de répartition ZR.

Ainsi, les connecteurs 7 qui servent à relier le boitier 2 au capteur 3 sont réparties selon cette zone de répartition. La zone de répartition ZR est polygonale ou de forme ovoïde. Elle peut être délimitée par un rectangle ou un cercle ou une ellipse ou un ovale, etc.

De cette manière, les forces exercées par les connecteurs sont compensées et la connexion électrique entre le boitier 2 et le capteur 3 est optimisée et faiblement bruitée.

Avec une telle répartition il est possible de prévoir un grand nombre de connecteurs 7 tout en limitant le bruit de mesure.

De manière avantageuse, six à vingt connecteurs 7 sont prévus, de préférence dix ou douze connecteurs.

A tire d'exemple, le bracelet exerce une force optimale au centre du boitier 2. Cette force est par exemple de l'ordre de 20 N ± 4 N (dépendante de nombreux paramètres). Cette force se réduit plus on s'éloigne du centre du boitier 2 (environ 18 N à 3mm du centre, environ 16 N à 5mm, environ 14 N à 7mm etc.). En outre, plus le nombre de connecteurs est important dans une zone serrée, plus leur force se cumule et s'oppose à la force du bracelet dans cette zone.

On considère à titre d'exemple que chaque broche pogo exerce une force de 1N qui s'oppose à celle exercée par le bracelet.

Si on dispose dans ce cas les pogos uniquement d'un seul côté (par exemple, douze pogo d'un côté) alors on a environ 12 N d'un seul côté, et donc le bracelet exerce sa force de 20N à 14N ±4 N (les connecteurs n'étant pas au centre du boitier) qui ne réussit pas à compenser suffisamment pour forcer un appui des pogos constant. La résultante est une perte de contact sur certain connecteur ressort lors de choc ou vibration du poignet.

Par contre, si l'on dispose six connecteurs ressorts autour du centre du boitier 2, on obtient environ 6N de chaque côté, force compensée par le bracelet qui est de 20 N à 14 N ±4 N.

Aussi, quelques connecteurs peuvent être isolés car ils représentent donc une force faible.

Également de manière avantageuse, les connecteurs 7 sont reliés entre eux pour effectuer les mesures, un connecteur étant associé à une microaiguille de sorte que les connexions des connecteurs reflètent celles prévues pour les microaiguilles.

Or si on relie entre eux trop de connecteurs ces derniers deviennent sensibles au bruit induit par les mouvements du poignet ou la connexion fluctuante du bracelet. Dès lors on prévoit de relier les connecteurs entre eux par paquets de deux à sept connecteurs, de préférence trois à quatre connecteurs.

De manière préférée, les connecteurs 7 sont reliés deux par deux et des mesures simultanées via ces paires de connecteurs sont effectuées.

Selon un mode de réalisation, les parties males sont réparties sur la face inférieure du boitier comme visible sur les figures 1, 2 et 3.

Selon un autre mode de réalisation, les parties males sont réparties sur la face supérieure du capteur (mode de réalisation non représenté).

On décrit ci-après des exemples de répartition.

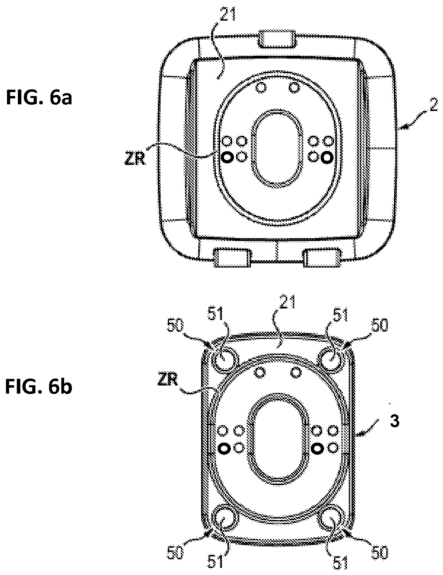

Les **figures 6a** et **6b** illustrent respectivement en vue de dessous le boitier 2 et en vue de dessus le capteur 3. Ces figures illustrent une répartition des connecteurs symétriques dans une zone ZR qui est ici un disque. Sur la figure 6a, les cercles représentent par exemple les parties males tandis que sur la figure 6b les cercles représentent les parties femelles. Sur ces figures huit connecteurs sont répartis symétriquement sur la zone ZR, sur chaque moitié il y a un nombre identique de connecteurs.

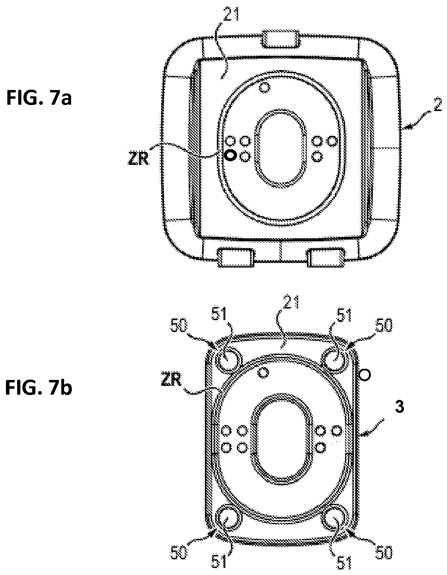

Les **figures 7a** et **7b** illustrent une autre répartition des connecteurs. Sur ces figures huit connecteurs sont réparties sur la zone ZR, quatre connecteurs étant sur une moitié, trois étant sur l'autre, un connecteur est en dehors de la zone de symétrie, six connecteurs sur huit sont répartis symétriquement.

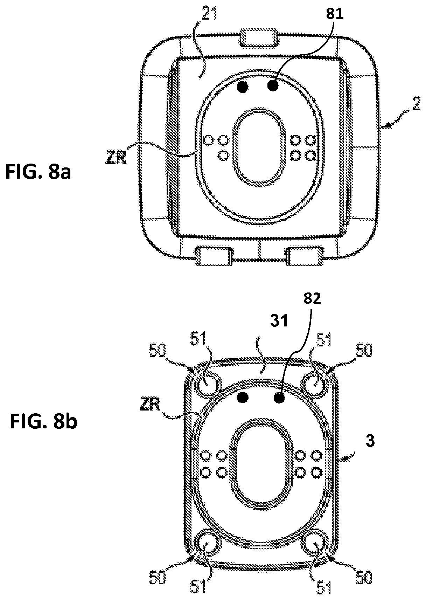

De manière complémentaire, comme décrit sur les **figures 8a** et **8b****,** des connecteurs additionnels peuvent être prévus (ci-après contacts) et comprennent aussi une partie male et/ou une partie femelle. Ils sont de technologie identique à celles des connecteurs pour le contact électrique entre le capteur et le boitier. Ces contacts ne sont pas prévus pour la connexion du boitier 2 au capteur 3 mais sont prévus pour par exemple recharger le boitier, ou pour connecter le capteur 3 à un système de test externe. Pour qu'ils ne gênent pas la répartition des forces ainsi décrite, on prévoit que la partie femelle associée au contact male soit un orifice qui empêche ainsi tout effort gênant entre le boitier et le connecteur. Dans le cas d'une broche pogo on peut prévoir dans ce cas un orifice plus grand que les parties femelles des connecteurs dédiés à la liaison entre le boitier et le capteur.

Les figures 8a et 8b illustrent par exemple des parties males et femelle (cercles vides) des connecteurs utile à la liaison boitier capteur et des parties mâles et femelles (cercles noirs) référencés par 81, 82 inutiles a cette liaison (pour les contacts). Si les parties males 81 sont prévus sur le boitier 2 alors les parties femelles 82 en face sont des orifices telles que les parties males s'enfoncent simplement dans les orifices sans exercer d'effort supplémentaire.

Sur les modes de réalisation des figures 6a, 6b, 7a, 7b il y a autant de parties males que de parties femelles mais on peut prévoir que cela ne soit pas le cas. Par exemple, comme on peut le voir sur les figures 8a, 8b en supposant que les parties femelles (cercles vides) sont sur le capteur, il y a plus de partie femelle que de partie male. Dès lors on peut prévoir que les parties males et les parties femelles de chaque connecteur sont réparties de manière identique et complémentaire sur leur face respective ou bien qu'elles ne soient pas réparties de manière identique et complémentaire sur leur face respective.

De manière complémentaire, une liaison amovible 50 entre le capteur et le boitier permet d'améliorer la force exercée par les moyens d'attache (le bracelet) et améliorer la liaison entre le capteur et le boitier. Cette liaison amovible est par exemple constituée par des parties aimantées complémentaires agencées sur le capteur et le boitier ou bien par des aimants 51 disposés par exemple sur le capteur 3, le boitier 2 étant alors métallique ou magnétique.

Cette liaison supplémentaire permet d'augmenter la force mécanique entre le boitier 2 et le capteur 3 et ainsi d'accroitre le nombre de connecteurs.

## Revendications

1. Dispositif de surveillance corporelle, comprenant :
- un boitier (2) comprenant une face inférieure (21) ;
- un capteur (3) comprenant une face inférieure (31) destinée à être disposé sur un membre d'un utilisateur et une face supérieure (32) disposée en face de la face inférieure (21) du boitier (2), le capteur et le boitier étant séparable l'un de l'autre à la demande, aucune liaison mécanique ne retenant le boitier (2) au capteur (3) fermement ensemble le boitier (2) étant en contact électrique et physique de manière lâche avec le capteur (3) par l'intermédiaire d'une pluralité de connecteurs (7), chaque connecteur (7) comprenant une partie femelle (71) et une partie male ressort (72) de manière à ce que la partie male (72) exerce une force sur la partie femelle (71) tendant à écarter le boitier (2) du capteur (3) ou inversement le dispositif comprenant en outre
- des moyens d'attache (23) du boitier (2) à un membre de l'utilisateur, lesdits moyens d'attache étant configurés pour appliquer une force de serrage afin de compenser la force écartant le boitier (2) du capteur (3) lorsque celui-ci est attaché au membre de l'utilisateur ;
le dispositif étant tel que les connecteurs sont répartis par rapport à une zone (ZR) de répartition définie sur la face supérieure (32) du capteur ou sur la face inférieure du boitier (31), plus de 70% des connecteurs étant répartis de manière symétrique dans chaque moitié de la zone de répartition (ZR) par rapport au centre du boitier qui correspond à l'épicentre de la force exercée par les moyens d'attache (23).

2. Dispositif selon la revendication 1, comprenant un patch adhésif (4) adapté pour être collé à la peau, le patch (4) supportant le capteur et permet de détacher le boitier (2) sans enlever le capteur (3).

3. Dispositif selon l'une des revendications précédentes, dans lequel le capteur (3) est configuré pour mesurer une grandeur physiologique ou biochimique de l'utilisateur, et comprend au moins une microaiguille adaptée pour être insérée fans la peau de l'utilisateur.

4. Dispositif selon l'une des revendications précédentes, dans lequel le capteur (3) s'engage dans une cavité (21) du boitier (2) située sur sa face destinée à être en contact avec la peau de l'utilisateur.

5. Dispositif selon l'une des revendications précédentes, dans lequel les parties males (72) et les parties femelles (71) de chaque connecteur sont réparties de manière identique et complémentaire sur leur face respective.

6. Dispositif selon l'une des revendications précédentes, dans lequel les parties males (72) et les parties femelles (71) de chaque connecteur ne sont pas réparties de manière identique et complémentaire sur leur face respective.

7. Dispositif selon l'une des revendications précédentes, comprenant six à vingt connecteurs, de préférence dix ou douze connecteurs répartis dans la zone de répartition (ZR), la zone de répartition (ZR) étant polygonale ou de forme ovoïde par exemple délimitée par un cercle, une ellipse ou un ovale, ou est une portion d'un polygone.

8. Dispositif selon l'une des revendications précédentes, comprenant en outre des zones (81, 82) comprenant une partie male et une partie femelle, la partie femelle étant telle que lorsque la partie male est introduite dans la partie femelle, la partie male n'exerce pas de force contre la partie femelle.

9. Dispositif selon l'une des revendications précédentes, dans lequel les connecteurs (7) sont reliés entre eux par groupe de deux à sept connecteurs, de préférence trois à quatre connecteurs.

10. Dispositif selon l'une des revendications précédentes, dans lequel chaque connecteur (7) comprend une partie femelle formée dans l'épaisseur du capteur (3) et une partie male (72) en saillie de la face inférieure (31) du boitier (2).

11. Dispositif selon l'une des revendications précédentes, dans lequel les parties males (72) sont des broches pogos configurées pour autoriser une amplitude comprise entre 50 µm et 2mm inclus lorsque le capteur (3) est en contact avec le boitier (2).

12. Dispositif selon l'une des revendications précédentes, comprenant une liaison (50) amovible entre le capteur (3) et le boitier (2).

13. Dispositif selon la revendication 12, dans lequel la liaison (50) est constituée par des parties aimantées complémentaires agencées sur le capteur et/ou le boitier.

14. Dispositif selon l'une des revendications précédentes, dans lequel le boitier (2) est en forme de boitier de montre et comprend des moyens d'attaches (23) du dispositif au poignet d'un utilisateur.

## Patentansprüche

1. Körperüberwachungsvorrichtung, umfassend:
- ein Gehäuse (2), das eine Unterseite (21) umfasst;
- einen Sensor (3), der eine Unterseite (31) umfasst, die dazu bestimmt ist, auf einer Gliedmaße eines Benutzers angeordnet zu sein, und eine Oberseite (32), die gegenüber der Unterseite (21) des Gehäuses (2) angeordnet ist, wobei der Sensor und das Gehäuse auf Wunsch voneinander trennbar sind, wobei keine mechanische Verbindung das Gehäuse (2) mit dem Sensor (3) fest zusammenhält,
wobei das Gehäuse (2) mit dem Sensor (3) über eine Vielzahl von Verbindern (7) lose in elektrischem und physischem Kontakt steht, wobei jeder Verbinder (7) einen Buchsenteil (71) und einen federnden Steckteil (72) umfasst, so dass der Steckteil (72) eine Kraft auf den Buchsenteil (71) ausübt, die dazu neigt, das Gehäuse (2) vom Sensor (3) zu entfernen oder umgekehrt; wobei die Vorrichtung ferner umfasst
- Mittel zur Befestigung (23) des Gehäuses (2) an einer Gliedmaße des Benutzers, wobei die Befestigungsmittel ausgelegt sind, um eine Klemmkraft auszuüben, um die Kraft auszugleichen, die das Gehäuse (2) vom Sensor (3) entfernt, wenn dieser an der Gliedmaße des Benutzers befestigt ist;
wobei die Vorrichtung so beschaffen ist, dass die Verbinder in Bezug auf einen definierten Verteilungsbereich (ZR) auf der Oberseite (32) des Sensors oder auf der Unterseite des Gehäuses (31) verteilt sind, wobei mehr als 70% der Verbinder symmetrisch in jeder Hälfte des Verteilungsbereichs (ZR) in Bezug auf die Mitte des Gehäuses verteilt sind, die dem Epizentrum der von den Befestigungsmitteln (23) ausgeübten Kraft entspricht.

2. Vorrichtung nach Anspruch 1, umfassend ein Haftpatch (4), das zum Kleben auf der Haut geeignet ist, wobei das Patch (4) den Sensor trägt und ermöglicht, das Gehäuse (2) abzunehmen, ohne den Sensor (3) zu entfernen.

3. Vorrichtung nach einem der vorhergehenden Ansprüche, wobei der Sensor (3) ausgelegt ist, um eine physiologische oder biochemische Größe des Benutzers zu messen, und mindestens eine Mikronadel umfasst, die geeignet ist, in die Haut des Benutzers eingeführt zu sein.

4. Vorrichtung nach einem der vorhergehenden Ansprüche, wobei der Sensor (3) in eine Ausnehmung (21) des Gehäuses (2) eingreift, die sich auf seiner Seite befindet, die dazu bestimmt ist, mit der Haut des Benutzers in Kontakt zu sein.

5. Vorrichtung nach einem der vorhergehenden Ansprüche, wobei die Steckteile (72) und die Buchsenteile (71) jedes Verbinders auf ihrer jeweiligen Seite identisch und komplementär verteilt sind.

6. Vorrichtung nach einem der vorhergehenden Ansprüche, wobei die Steckteile (72) und die Buchsenteile (71) jedes Verbinders auf ihrer jeweiligen Seite nicht identisch und komplementär verteilt sind.

7. Vorrichtung nach einem der vorhergehenden Ansprüche, die sechs bis zwanzig Verbinder umfasst, vorzugsweise zehn oder zwölf Verbinder, die im Verteilungsbereich (ZR) verteilt sind, wobei der Verteilungsbereich (ZR) polygonal oder eiförmig ist, beispielsweise durch einen Kreis, eine Ellipse oder ein Oval begrenzt, oder ein Teil eines Polygons ist.

8. Vorrichtung nach einem der vorhergehenden Ansprüche, die ferner Bereiche (81, 82) umfasst, die einen Steckteil und einen Buchsenteil umfassen, wobei der Buchsenteil so beschaffen ist, dass der Steckteil keine Kraft gegen den Buchsenteil ausübt, wenn der Steckteil in den Buchsenteil eingeführt ist.

9. Vorrichtung nach einem der vorhergehenden Ansprüche, wobei die Verbinder (7) in Gruppen von zwei bis sieben Verbinder, vorzugsweise drei bis vier Verbinder, miteinander verbunden sind.

10. Vorrichtung nach einem der vorhergehenden Ansprüche, wobei jeder Verbinder (7) einen in der Dicke des Sensors (3) ausgebildeten Buchsenteil und einen aus der Unterseite (31) des Gehäuses (2) herausragenden Steckteil (72) umfasst.

11. Vorrichtung nach einem der vorhergehenden Ansprüche, wobei die Steckteile (72) Kontaktstifte sind, die ausgelegt sind, um eine Amplitude zwischen 50 µm und 2 mm einschließlich zulassen, wenn der Sensor (3) mit dem Gehäuse (2) in Kontakt ist.

12. Vorrichtung nach einem der vorhergehenden Ansprüche, die eine lösbare Verbindung (50) zwischen dem Sensor (3) und dem Gehäuse (2) umfasst.

13. Vorrichtung nach Anspruch 12, wobei die Verbindung (50) aus komplementären magnetisierten Teilen besteht, die am Sensor und/oder am Gehäuse eingerichtet sind.

14. Vorrichtung nach einem der vorhergehenden Ansprüche, wobei das Gehäuse (2) die Form eines Uhrengehäuses hat und Mittel (23) zum Befestigen der Vorrichtung am Handgelenk eines Benutzers umfasst.

## Claims

1. Body, monitoring device comprising:
- a case (2) comprising a lower face (21);
- a sensor (3) comprising a lower face (31) intended to be arranged on a user's limb and an upper face (32) arranged opposite the lower face (21) of the housing (2), the sensor and the housing being separable from each other as required, no mechanical connection holding the housing (2) to the sensor (3) firmly together,
the housing (2) being in loose electrical and physical contact with the sensor (3) via a plurality of connectors (7), each connector (7) comprising a female part (71) and a spring male part (72) so that the male part (72) exerts a force on the female part (71) tending to move away the housing (2) from the sensor (3) or vice versa; the device further comprising:
- means for attaching (23) the case (2) to a limb of the user, said attaching means being configured to apply a clamping force in order to compensate for the force moving away the case (2) from the sensor (3) when the latter is attached to the member of the user;
the device being such that the connectors are distributed with respect to a distribution zone (ZR) defined on the upper face (32) of the sensor or on the lower face of the casing (31), more than 70% of the connectors being distributed symmetrically in each half of the distribution zone (ZR) with respect to the centre of the casing, which corresponds to the epicentre of the force exerted by the attachment means (23).

2. The device as claimed in claim 1, comprising an adhesive patch (4) adapted to be glued to the skin, the patch (4) supporting the sensor and allowing the case (2) to be detached (2) without removing the sensor (3).

3. The device as claimed in one of the preceding claims, wherein the sensor (3) is configured to measure a physiological or biochemical quantity of the user and comprises at least one micro-needle adapted to be inserted into the user's skin.

4. The device as claimed in one of the preceding claims, wherein the sensor (3) engages in a cavity (21) in the case (2) on its side intended to come into contact with the user's skin.

5. The device as claimed in one of the preceding claims, wherein the male parts (72) and the female parts (71) of each connector are identically and complementarily distributed on their respective faces.

6. The device as claimed in one of the preceding claims, wherein the male parts (72) and the female parts (71) of each connector are not distributed identically and complementarily on their respective faces.

7. The device as claimed in one of the preceding claims, comprising six to twenty connectors, preferably ten or twelve connectors distributed in the distribution zone (ZR), the distribution (ZR) being zone polygonal or ovoid in shape, for example delimited by a circle, an ellipse or an oval, or is a portion a polygon.

8. The device as claimed in one of the preceding claims, further comprising zones (81, 82) comprising a male part and a female part, the female part being such that when the part male is introduced inserted into the into female, the male part is not exerts any force against the female part.

9. The device as claimed in one of the preceding claims, wherein the connectors (7) are linked together in groups of two to seven connectors, preferably three to four connectors.

10. The device as claimed in one of the preceding claims, wherein each connector (7) comprises a female part formed in the thickness of the sensor (3) and a male part (72) projecting from the lower face (31) of the casing (2).

11. The device as claimed in one of the preceding claims, wherein the male parts (72) are pogos pins configured to allow an amplitude of between 50 µm and 2 mm inclusive when the sensor (3) is in contact with the casing (2).

12. The device as claimed in one of the preceding claims, comprising a link (50) removable between the sensor (3) and the housing (2).

13. The device as claimed in one of the preceding claims, wherein the connection (50) is constituted by complementary magnetic parts arranged on the sensor and/or the casing.

14. The device as claimed in one of the preceding claims, wherein the case (2) is in the form of a watch case and comprises means (23) for attaching the device to a user's wrist.
